# EUROPEAN PATENT APPLICATION

(11) **EP 1 746 417 A1**
(43) Date of publication of application: **24.01.2007**
(21) Application number: 05015998.7
(22) Date of filing: 22.07.2005
(51) Int. Cl.: G01N 33/38, B07C 5/342

(54) **Method and device for investigating a mineral sample**

(71) Applicant: GSF-FORSCHUNGSZENTRUM FÜR UMWELT UND GESUNDHEIT, GMBH, 85764 Neuherberg (DE)
(72) Inventor: Göksu, H. Yeter, 80805 München (DE); Fasoli, Mauro, 80807 München (DE)
(74) Representative: Hertz, Oliver

(57) **Abstract**

A method of investigating a mineral sample (2) comprises the steps of carrying out at least one thermoluminescence measurement with the mineral sample (2), determining a thermoluminescence parameter derived from a result of the thermoluminescence measurement, and evaluating the thermoluminescence parameter for classifying the mineral sample (2) as being artificially coloured or naturally coloured. Furthermore, an investigation device (1) for investigating a mineral sample is described.

## Description

### Field of the invention

This invention relates to a method and a device for investigating a mineral sample, in particular for classifying the mineral sample as being artificially coloured or naturally coloured.

### Technical Background

Commercial use of radiation treatment for alteration of the colour of gemstones is a known practice. It is know that certain manufacturers offer large amounts of cheap, naturally colourless stones, which have been exposed to high doses of radiation, as naturally coloured stones resulting in an enhanced profit. The higher price of naturally coloured gemstones is due to the fact, that they are formed in nature only at certain environmental conditions over a long period of time, wherein a long time exposure to irradiation over millions of years is necessary.

Artificial coloration of gemstones can easily be obtained by treating the gemstones e.g. in gamma ray facilities, linear accelerators or nuclear reactors, wherein merely a short time exposure, e.g. a few hours, is sufficient. The colour of artificially irradiated gemstones cannot be visually distinguished from the colour of naturally irradiated gemstones.

### Object of the invention

It is an object of the invention to provide a cheap and easy method and device allowing a reliable decision whether a mineral sample, in particular a gemstone is artificially coloured or naturally coloured.

This object is solved with a method according to claim 1 and a device according to claim 15. Preferred embodiments of the invention are defined in the dependent claims.

### Summary of the invention

The invention makes use of the fact, that the coloration of mineral, in particular gemstones is caused by trapped charges, wherein the trapped charges have been produced by an energy input due to irradiation. The trapped charges (or: trapped electrons) have a decay time so that naturally coloured gemstones having lost a part of the trapped electrons , in particular at shallow or intermediate energy levels can be distinguished from artificially coloured gemstones.

With the invention a discrimination is carried out using the thermoluminescence parameter whether the mineral sample has been artificially irradiated or naturally irradiated. For example, an integrated light intensity indicating an amount of mineral sample luminescence above a certain discrimination threshold is used to determine an artificially colouration of the mineral sample. An artificially colouration caused by an artificially irradiation results in a considerable higher amount of emitted light during heating in the thermoluminescence measurement due to the fact that many unstable trapped electrons not have decayed at the moment of measurement.

Throughout this description the terms "naturally" and "artificially" serve to differentiate between different irradiating processes causing the colour of the mineral sample, e.g. a smoky brown coloured gemstone. Herein the term "naturally coloured" is used for mineral samples, which have been exposed to a natural irradiation, which caused the colouration of the mineral sample. On the other hand, "artificially coloured" means that the colouration is caused by a short term and powerful irradiation, e.g. a few hours or days in an irradiating facility.

The invention uses luminescence measurements of the mineral sample, e.g. a gemstone, to determine a thermoluminescence parameter. The thermoluminescence parameter can comprise e.g. a shape of a glow curve or an integrated glow curve value (integrated light intensity), which glow curve indicates the emitted light from the mineral sample during heating of the mineral sample. For example, the mineral sample can be heated with 5 K/s and simultaneously the mineral sample luminescence is sensed by a luminescence sensor, e.g. a photomultiplier or a photodiode. The mineral sample can also be heated with other heating rates, wherein heating rates between 1 K/s and 10 K/s are preferred. Higher heating rates are advantageous for fast measurement. Lower heating rates provide a better resolution.

If according to a preferred embodiment of the invention the thermoluminescence parameter is represented by a curve shape of a glow curve of the mineral sample luminescence, the thermoluminescence parameter can be given simply by the fact whether the glow curve of the mineral sample luminescence has a maximum or not. Advantageously, the measurement and classification of the sample is very easy to be provided. In particular, the artificially coloured mineral sample can be found if the glow curve does not include a maximum due to a masking shape of the glow curve.

Alternatively, the step of determining the thermoluminescence parameter may comprise the step of integrating a glow curve of the mineral sample luminescence for obtaining an integrated light intensity representing the thermoluminescence parameter. This embodiment has particular advantages in terms of reliability and reproducibility. According to this embodiment, even artificially coloured mineral samples can be detected which have been subjected to a heating or any other treatment.

In a preferred embodiment of the invention the thermoluminescence measurement on the mineral sample and the determination of a thermoluminescence parameter is carried out repeatedly, wherein at least two luminescence parameters, e. g. two intensities of luminescence radiation, in two separate thermoluminescence measurements are determined.

Before a thermoluminescence measurement preferably a pre-irradiation process is carried out using a radiation source. By this means the mineral sample is artificially irradiated, so that whole trapping centres are activated which would lead to a considerable sensitisation of the luminescence efficiency. A subsequent heating to a high temperature, e.g. between 400°C and 700°C, thermally moves holes from reservoirs to luminescence centres leading to an increase in radiation sensitivity for electrons at lower energy levels. This effect causes an increase in the emitted light during heating especially if the mineral sample is naturally coloured so it is possible to determine an artificial colouration.

Hence, according to a preferred embodiment of the invention a radiation source is used which is preferably mounted in a receptacle of the sample and which can be controlled by an operator. In particular, the radiation source is removable from the receptacle, so that the radiation source after use can be stored in a led container. A preferred radiation source is a beta radiation source, e.g. a ⁹⁰Sr beta radiation source.

In a preferred embodiment of the invention the mineral sample is pre-heated to a pre-heating temperature in a pre-heating process before the thermoluminescence measurement is carried out. It is especially preferred, that the pre-heating process is carried out in combination with the pre-irradiation process, wherein a pre-heating process is able to activate whole trapping centres. The pre-heating process can be carried out in any combination with the pre-irradiation process and in any combination with the thermoluminescence measurement, wherein it is preferred that firstly the mineral sample is pre-heated, secondly it is treated in a pre-irradiation process and thirdly it is investigated in a luminescence measurement.

Furthermore, it is preferred, that the aforementioned series of pre-heating, pre-irradiation and investigation is carried out repeatedly, wherein different temperatures and pre-irradiation doses are used. For example, a first series can be carried out with a maximum temperature of 150°C and a pre-irradiation dose of 40 mGy and the second series is carried out with a pre-heating temperature of 500°C, a thermoluminescence measurement maximum temperature of 150°C and a pre-irradiation dose of 40 mGy.

A further possibility of combining the aforementioned investigation steps is to divide a mineral sample in two aliquots which are identical but treated differently. For example, one of the two identical aliquots can be pre-irradiated with a relatively high dose of 30 kGy and subsequently pre-heated and investigated in a thermoluminescence measurement, wherein the other aliquot is not pre-irradiated. The relatively high pre-irradiation before the pre-heating has a considerable impact on the thermoluminescence properties of a naturally coloured mineral sample. However, an artificially coloured mineral sample shows merely a slight change in the thermoluminescence properties. This is due to the fact that the artificially coloured mineral sample has been treated in a high-dosed radiation treatment before, namely in the artificial colouration process.

The mineral sample is preferably grinded before the luminescence measurement is carried out. This is advantageous because free-surface effects of the mineral sample are equalized. Especially preferred is a grain size of 90 µm to 150 µm.

Mineral samples which can be investigated in a process or device according to the invention include quartz and gemstones, e. g. diamonds. However, the invention is not limited to these mineral samples. All artificially colourable mineral samples can be investigated using the invention.

According to the invention there is provided a device for investigating the mineral sample, comprising a receptacle for receiving the mineral sample, wherein the receptacle includes a heating device and a luminescence sensor. The heating device preferably heats the whole internal space of the receptacle thereby heating the mineral sample uniformly. Furthermore, the device comprises an evaluating device, which is connected to the luminescence sensor and is adapted to evaluate the thermoluminescence parameter. The luminescence sensor preferably comprises a photomultiplier, which has advantages in terms of direct measurement of light intensities. The evaluating device can be a personal computer with an interface for the signal of the luminescence sensor.

A preferred embodiment of the invention comprises an integration device for accumulating or integrating the intensity of the mineral sample luminescence. Due to the decay time of trapped charges, artificially coloured mineral samples tend to emit more light during the thermoluminescence measurement compared to naturally coloured mineral samples. Hence, a simple accumulation of the mineral sample luminescence can be sufficient to decide whether the mineral sample is naturally coloured or artificially coloured.

A preferred device according to the invention comprises a memory which is adapted to store thermoluminescence parameters of thermoluminescence measurements, in particular light intensities during heating or light intensities of multiple measurements. This is advantageous due to the possibility of comparing two or more thermoluminescence parameters of two or more subsequent thermoluminescence measurements. For example, due to the more stable electron traps of a naturally coloured mineral sample, this sample tends to show a smaller difference in the emitted light of two subsequent measurements. Therefore, the device according to the invention preferably comprises a comparing device for comparing at least two thermoluminescence parameters, e. g. glow curves, for allowing a classification of the mineral sample.

Another subject of the invention is the use of a device according to the invention for analysing a mineral sample.

### Brief description of the drawings

The invention is further explained in the following detailed description of the drawings which are not tended to limit the invention outlined by the appended claims.
- Figure 1: is a schematic drawing of a device according to the invention comprising a receptacle shown in a front sectional view and an evaluating device.
- Figure 2: is a flow chart of a first part of a process according to the invention which can be carried out with the device shown in Figure 1.
- Figure 3: is a flow chart of a second part of the process partly shown in Figure 2.
- Figures 4 to 7: are diagrams showing results of luminescence measurements with artificially or naturally coloured quartz samples.
- Figure 8: is another diagram showing thermoluminescence measurements carried out on a naturally coloured quartz.

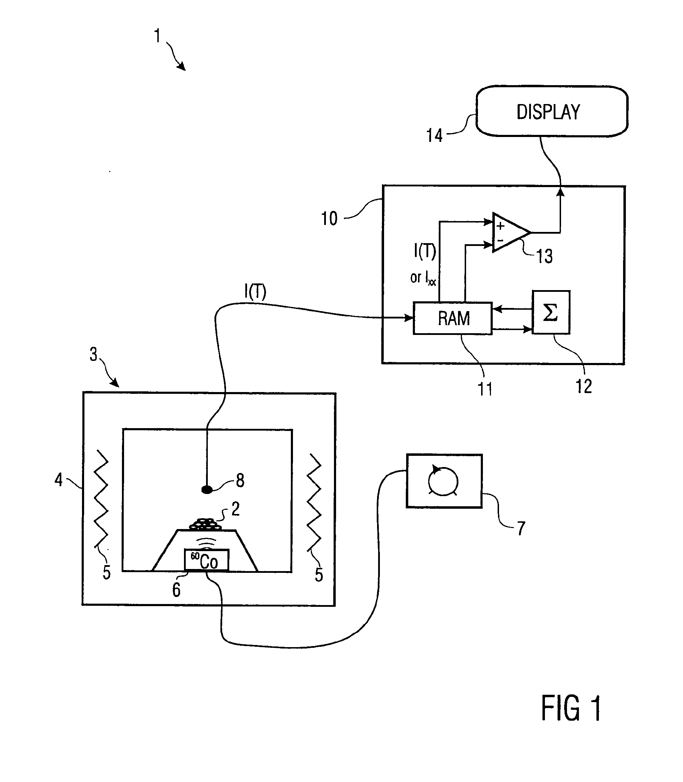

In Figure 1, an investigation device 1 according to the invention is shown which can be used to investigate a mineral sample 2. The grinded mineral sample 2 (grain size approximately 100 µm) is arranged in a receptacle 3. The receptacle 3 comprises a body frame 4 in which a heating device 5 is comprised. The heating device 5 can be controlled manually or automatically by a control device (not shown). Furthermore, the receptacle comprises a ⁹⁰Sr beta radiation source 6, which is used to irradiate the mineral sample 2. The beta radiation 6 is controlled manually by a radiation control device 7. Preferably, the receptacle or any mount holding the sample during heating is as small as possible, e.g. smaller than 1 cm, for improving the control of the heating rate.

The intensity I of the light, which is emitted by the mineral sample 2 during a thermoluminescence measurement is sensed by a luminescence sensor 8 (e.g. a photomultiplier) which is coupled to an evaluating device 10. The evaluating device also records the temperature T of the mineral sample 2, wherein the temperature T can be evaluated by a common temperature sensor (e.g. a thermocouple) arranged in the receptacle 3. The evaluating device 10 receives the signal of the luminescence sensor 8 and stores the data evaluated during a thermoluminescence measurement in a random access memory 11. The random access memory 11 is coupled to an integration device 12 which is adapted to accumulate the intensity of the mineral sample luminescence which is sensed by the luminescence sensor 8. On the other hand, the random access memory 11 not only stores the accumulated intensity but also stores the complete glow curve I(T) (Intensity I over temperature T) of a thermoluminescence measurement, i.e. the whole data evaluated during one heating cycle is stored.

The evaluating device 10 further comprises a comparing element 13 to compare the glow curves or accumulated intensities of mineral sample luminescence of two different thermoluminescence measurements. Also a comparison of two thermoluminescence measurements of different mineral samples or different aliquots of a mineral sample is possible. The result of the comparison allows a discrimination of the colouration process of the mineral sample and is transmitted to a display 14. The display 14 is used to display the result of the discrimination to an operator.

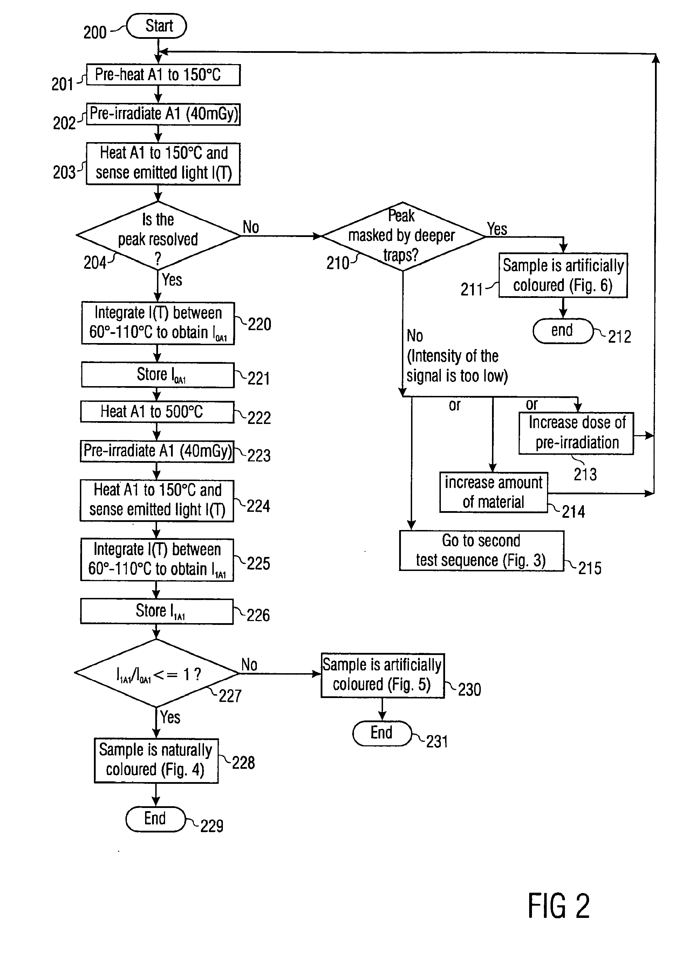

In Figure 2, a first part of a process according to the invention is shown which can be carried out with the investigation device shown in Figure 1. The process is started in step 200. In step 201 a mineral sample A1, e. g. a quartz, is pre-heated to 150°C with a heating rate of 5 K/s. The pre-heating or heating of mineral samples in the outlined process is always carried out with a heating rate of 5 K/s unless it is noted otherwise. Subsequently in step 202, the mineral sample A1 is pre-irradiated by a dose of 40 mGy. These steps of pre-heating and pre-irradiation serve as a sample preparation (reset), which is preferably implemented when the sample has been handled before the investigation under irreproducible thermal and irradiation conditions.

In step 203, the thermoluminescence measurement (measurement of the glow curve) is started by heating the mineral sample A1 to 150°C and sensing the mineral sample luminescence I(T). I(T) is the glow curve of the present luminescence measurement.

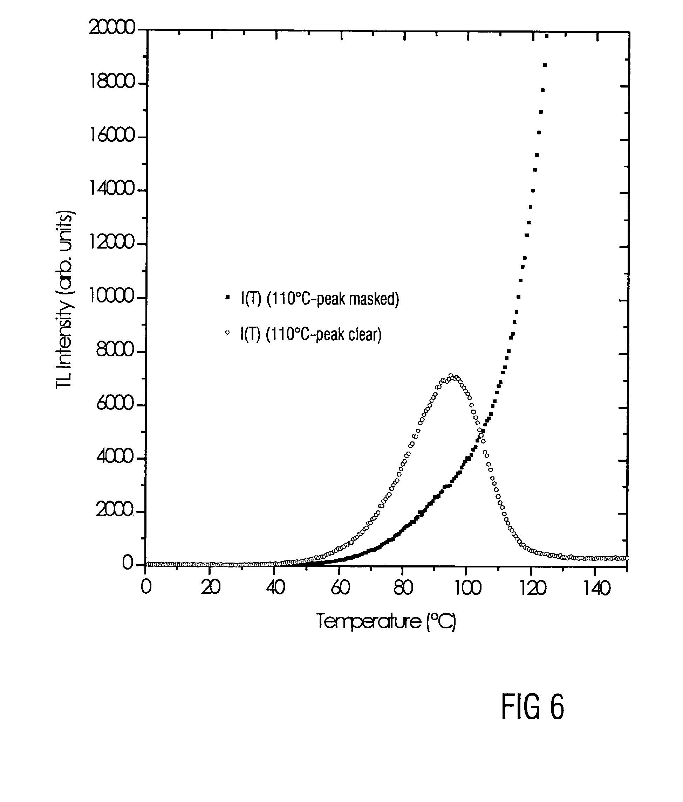

In a deciding step 204 it is checked, whether the glow curve I(T) allows to resolve a peak at about 110°C. If the 110°C-peak cannot be clearly resolved, it is asked in another deciding step 210, if the peak is masked (superposed) by deeper traps which means that the mineral sample comprises deeper electron traps caused by a high dose artificially radiation. Hence, if the peak is masked by deeper traps, it is decided, that the mineral sample A1 is artificially coloured (step 211) and the process is terminated (step 212). In Figure 6, two glow curves are depicted, wherein the solid dots belong to a glow curve in which the 110°C-peak is masked by deeper traps (quartz artificially coloured with a dose of 30 kGy).

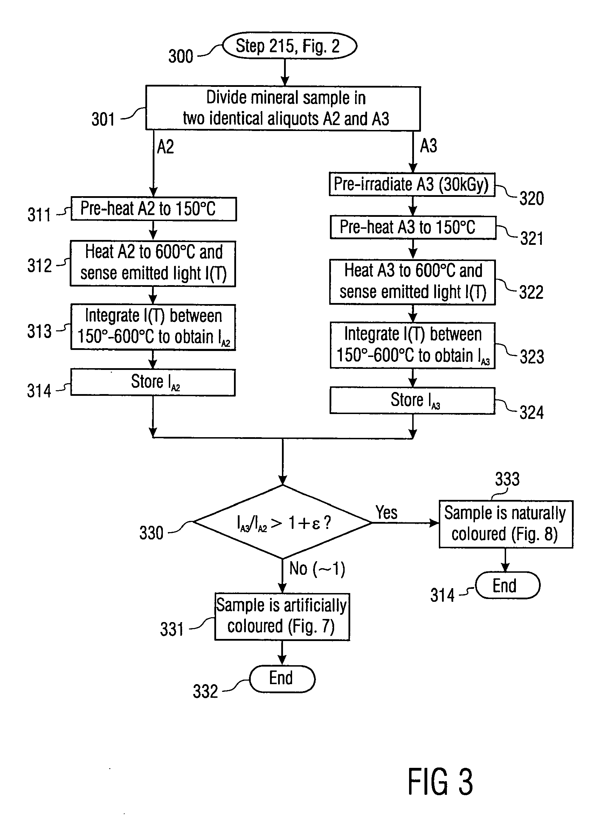

If it is determined in step 210 that the 110°C-peak is not masked by deeper traps but nevertheless the 110°C-peak is not clear due to a low intensity of the signal, the process is started again with an increased dose of pre-irradiation or an increased amount of sample material (step 213 or 214 respectively). Another possibility is to carry out another test sequence which is depicted in Figure 3.

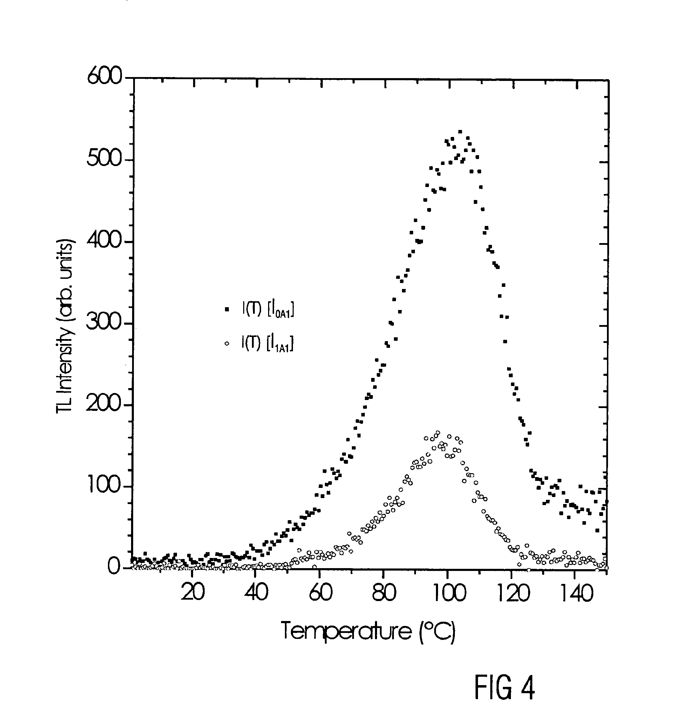

If the result of the deciding step 204 is a clear 110°C-peak, the glow curve I(T) is accumulated between 60°C and 110°C to obtain the accumulated intensity I_{0A1} (step 220). This value is stored in step 211. Subsequently the mineral sample A1 is pre-heated to 500°C in a step 212 and pre-irradiated with 40 mGy in step 223. Then, a second luminescence measurement is carried out, wherein the mineral sample A1 is heated to 150°C. Again, a glow curve I(T) of the sensed emitted light is recorded and integrated between 60°C and 110°C to obtain I_{1A1} in steps 224 and 225. The obtained value is stored, step 226. In a deciding step 227, the mineral sample is classified by comparing the two accumulated intensities I_{0A1} and I_{1A1}. If I_{1A1} is smaller or equal to I_{0A1}, then the process proceeds to step 228, i.e. it is determined that the mineral sample A1 is naturally coloured, and the process is terminated in step 229. Figure 4 shows an example of the two subsequent thermoluminescence measurements carried out on a naturally coloured mineral sample (quartz).

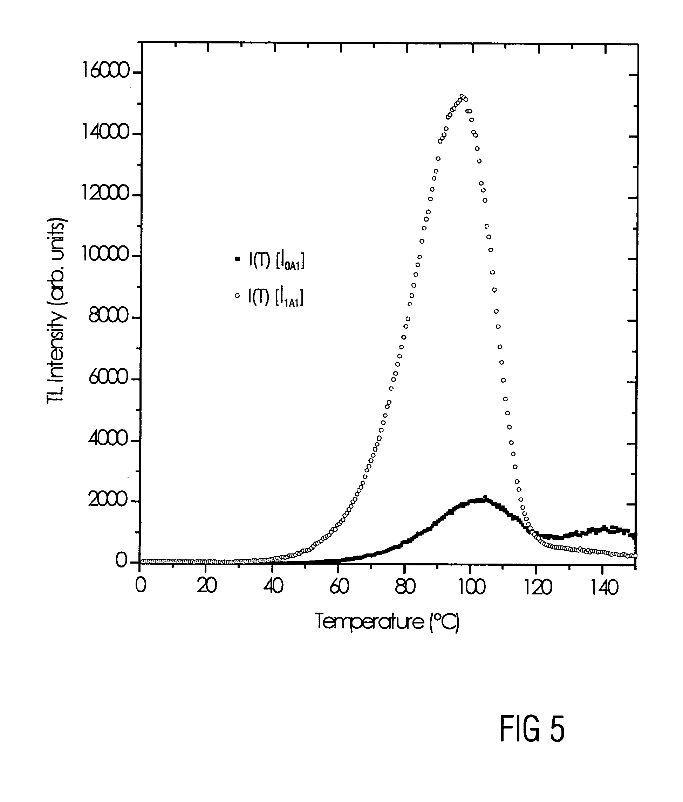

Otherwise, it is decided in step 227 that the mineral sample A1 is artificially coloured (step 230), and the process is terminated in step 231. Figure 5 shows an example of results for an artificially coloured mineral sample (quartz coloured with a dose of 30 kGy).

In Figure 3, a second test sequence is shown which can be carried out in case the results of the luminescence measurement of the process depicted in Figure 2 does not show a clear 110°C-peak. In a step 301 two identical aliquots A2 and A3 are formed by dividing the mineral test sample in two equal parts of grinded particles. The two aliquots A2 and A3 are investigated in two luminescence measurements. The only difference in treating the two aliquots A2 and A3 is a pre-irradiation treatment of the aliquot A3 with a relatively high beta radiation dose of 30 kGy (step 320). Besides this difference the two aliquots A2 and A3 are handled identical so the steps 311 to 314 for aliquot A2 correspond to the steps 321 to 324 for aliquot A3.

In detail, the aliquots are pre-heated to 150°C (steps 311 and 321 respectively), wherein the pre-heating process is subsequent to the pre-irradiation process for aliquot A3. Subsequently the aliquots are heated to 600°C (steps 312 and 322), wherein the emitted light is sensed and a glow curve I(T) is obtained for each of the aliquots. The glow curves are accumulated in steps 314 and 323 to obtain I_{A2} and I_{A3}, respectively. These values are stored in steps 314 and 324.

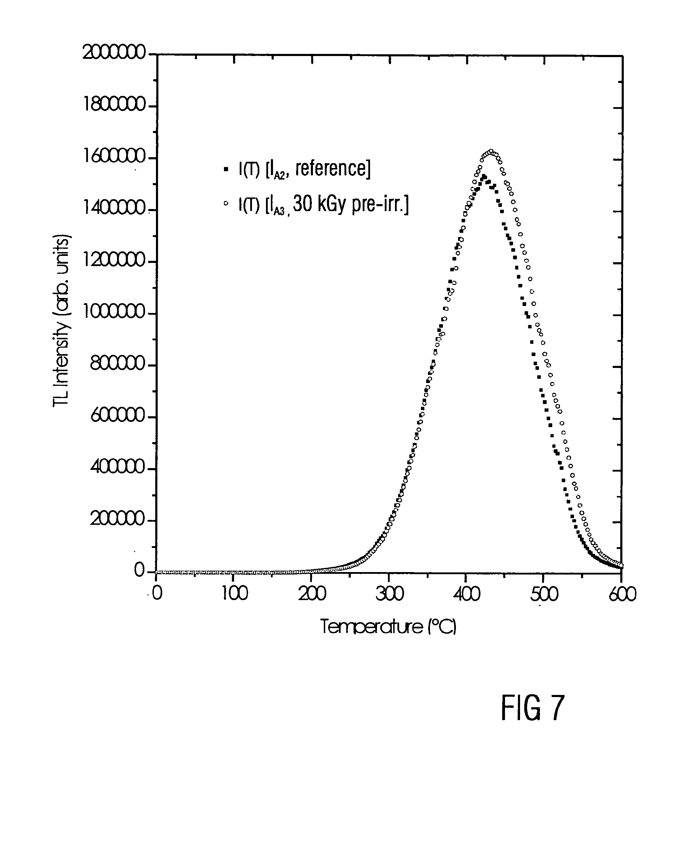

In a deciding step 330, it is tested if the two accumulated intensities I_{A2} and I_{A3} are roughly equal (example: ε = +/-0.2). If so, it is decided that the mineral sample is artificially coloured since the relatively high pre-irradiation dose of 30 kGy (step 320) did not alter the luminescence properties of the mineral sample. Hence, it is concluded that the mineral sample must have been irradiated before with a high-dose irradiation which is typically for artificial irradiation. Figure 7 shows typical results for this case (step 331). The glow curves of artificially coloured quartz before and after laboratory irradiation (30 kGy) are nearly the same. The process is terminated in step 332.

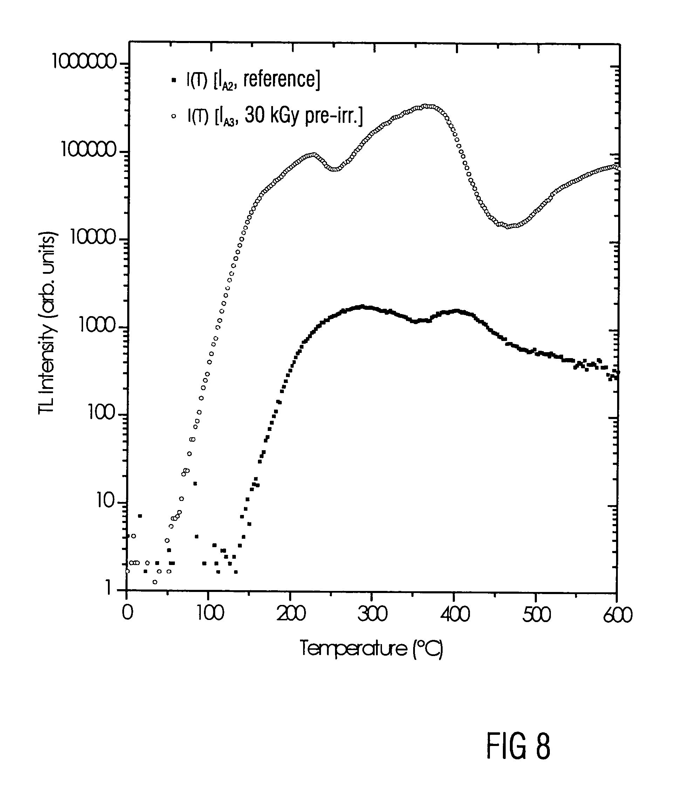

If in step 330 it is decided that the difference between the two accumulated intensities I_{A2} and I_{A3} is greater than a certain threshold, the mineral sample is considered as to be naturally coloured (step 333) and the process is terminated in step 334. Figure 8 shows typical test results for a naturally coloured mineral sample. The influence of the additional artificial irradiation can clearly be seen in the depicted glow curves.

The invention may be embodied in other specific forms without departing from the spirit or essential attributes thereof and, accordingly, reference should be made to the appended claims, in addition to the foregoing specification, as indicating the scope of the invention.

## Claims

1. Method of investigating a mineral sample (2) comprising the steps of:
(a) carrying out at least one thermoluminescence measurement with the mineral sample (2),
(b) determining a thermoluminescence parameter derived from a result of the thermoluminescence measurement, and
(c) evaluating the thermoluminescence parameter for classifying the mineral sample (2) as being artificially coloured or naturally coloured.

2. Method according to claim 1, wherein the thermoluminescence parameter is represented by a curve shape of a glow curve of the mineral sample luminescence and the step of determining the thermoluminescence parameter comprises the step of detecting whether the glow curve of the mineral sample luminescence has a maximum or not.

3. Method according to claim 2, wherein the step of evaluating the thermoluminescence parameter comprises the step of detecting whether the curve shape of the glow curve includes a masking of the maximum or not, and wherein the mineral sample (2) is classified as being artificially coloured if the glow curve includes a masking of the maximum.

4. Method according to claim 1 or 2, wherein the step of determining the thermoluminescence parameter comprises the step of integrating a glow curve of the mineral sample luminescence for obtaining an integrated light intensity representing the thermoluminescence parameter.

5. Method according to claim 4, wherein the step of evaluating the thermoluminescence parameter comprises the step of comparing the integrated light intensity with at least one reference intensity.

6. Method according to claim 5, wherein the series of steps (a) and (b) is carried out repeatedly with different measurement conditions for obtaining a plurality of integrated light intensities at least one of which being used as the reference intensity.

7. Method according to claim 6, wherein the series of steps (a) and (b) is carried out repeatedly with different pre-heating temperatures wherein the mineral sample (2) is classified as being artificially coloured if the integrated light intensity obtained with a higher pre-heating temperature is greater than the integrated light intensity obtained with a lower pre-heating temperature.

8. Method according to claim 6, wherein the series of steps (a) and (b) is carried out repeatedly with different pre-irradiating doses wherein the mineral sample (2) is classified as being artificially coloured if the integrated light intensities obtained with the different pre-irradiating doses have equal values.

9. Method according to at least one of the foregoing claims, comprising at least one step of preparing the mineral sample (2) before the series of steps (a) and (b) and/or between two series of steps (a) and (b), the preparing step including at least one of:
- pre-irradiating the mineral sample (2) using a radiation source (6), and
- pre-heating the mineral sample (2) to a pre-heating temperature.

10. Method according to claim 9, wherein the radiation source is a beta radiation source (6).

11. Method according to claim 9 or 10, wherein the pre-heating temperature is selected between 100°C and 200°C.

12. Method according to at least one of the foregoing claims, comprising a step of grinding the mineral sample (2) before carrying out the series of steps (a) and (b).

13. Method according to at least one of the foregoing claims, wherein the mineral sample (2) comprises a gemstone.

14. Method according to at least one of the foregoing claims, wherein the mineral sample (2) comprises diamond.

15. Device (1) for investigating a mineral sample (2), comprising:
- a receptacle (3) for receiving the mineral sample (2),
- a heating device (5) arranged for heating the mineral sample (2) in the receptacle (3),
- a luminescence sensor (8) adapted to sense mineral sample luminescence during heating of the mineral sample (2), and
- an evaluating device (10) connected with the luminescence sensor (8) and adapted for determining a thermoluminescence parameter to evaluate a thermoluminescence parameter and for evaluating the thermoluminescence parameter for classifying the mineral sample (2) as being artificially coloured or naturally coloured.

16. Device according to claim 15, wherein the evaluating device (10) comprises an integration device (12) for integrating the intensity of the mineral sample luminescence.

17. Device according to at least one of the claims 15 or 16, wherein the evaluating device (8) comprises
- a memory (11) adapted for storage of thermoluminescence parameters, and
- a comparing device (13) for comparing two stored thermoluminescence parameters of two subsequent thermoluminescence measurements.

18. Device according to at least one of the claims 15 to 17, further comprising
- a radiation source (6) which can be positioned in the receptacle (3).

19. Device according to claim 18, wherein the radiation source is a beta radiation source (6).

20. Use of a device (1) according to at least one of the claims 15 to 19 for investigating a mineral sample (2).
